# EUROPEAN PATENT SPECIFICATION

(11) **EP 4 452 074 B1**
(45) Date of publication and mention of the grant of the patent: **23.09.2026**
(21) Application number: 22840115.4
(22) Date of filing: 19.12.2022
(51) Int. Cl.: A61B 6/02, G01N 23/087, A61B 6/00, A61B 6/40, A61B 6/42

(54) **SPECTRAL X-RAY CT IMAGING SYSTEM**
SPEKTRALES RÖNTGEN-CT-ABBILDUNGSSYSTEM
SYSTÈME D'IMAGERIE SPECTRALE CT À RAYONS X

(30) Priority: 21.12.2021 EP 21216487
(43) Date of publication of application: 30.10.2024
(73) Proprietor: Koninklijke Philips N.V., 5656 AE Eindhoven (NL)
(72) Inventor: PROKSA, Roland, 5656 AG Eindhoven (NL); KOEHLER, Thomas, 5656 AG Eindhoven (NL); GRASS, Michael, 5656 AG Eindhoven (NL); WUELKER, Christian, 5656 AG Eindhoven (NL); WILD, Sebastian, 5656AG Eindhoven (NL)
(74) Representative: Philips Intellectual Property & Standards
(86) International application number: PCT/EP2022/086620
(87) International publication number: WO 2023/117884

(56) References cited:
- WO-A1-2021/094806
- CN-A- 101 897 595
- US-A1- 2010 303 196
- US-A1- 2021 007 691

## Description

### FIELD OF THE INVENTION

The present invention relates to a spectral X-ray CT imaging system, a spectral X-ray CT imaging method, as well as to a computer program element and a computer readable medium.

### BACKGROUND OF THE INVENTION

The stability of projection-based material decomposition in dual layer detector spectral CT imaging is most optimal if both energy channels in each projection measure approximately the same number of photons. This guarantees robustness in the decomposition process due to equal noise level per channel. However, in practical imaging this is hard to achieve since the tube voltage is selected prior to the scan and not modified during imaging. Due to changing patient size and the angular dependence of the line integrals variations in the signal strength per channel are unavoidable. X-ray tube voltages are not varied during imaging because the projections of a single scan would contain different contrast information caused by different X-Ray tube spectra. This becomes a more severe problem in spectral imaging where the spectrum must be known and kept constant in the material decomposition.

This leads to non-optimal material decomposition.

There is a need to resolve this issue.

US2010/303196A1 discloses modulation of the tube voltage and/or tube current in a dual layer detector CT imaging system according to attenuation in the subject portion being scanned to improve dose efficiency.

The invention is defined in the claims with further aspects in the dependent claims.

### SUMMARY OF THE DISCLOSURE

It would be advantageous to provide for improved projection-based material decomposition in dual layer detector spectral CT imaging. The object of the present invention is solved with the subject matter of the independent claims, wherein further embodiments are incorporated in the dependent claims. It should be noted that the following described aspects and examples of the invention apply to the spectral X-ray CT imaging system, the spectral X-ray CT imaging method as well as to the computer program element and the computer readable medium.

In a first aspect, there is provided a spectral X-ray CT imaging system according to claim 1.

**In** other words, rather than keep the X-ray tube voltage constant during a CT spectral energy scan the X-ray tube voltage is altered, if necessary, for each acquisition dependent upon the body portion projection. Thus, the effects of beam hardening can be accounted for, where the tube voltage can be adjusted to account for different thickness and/or attenuation profiles of the body portion.

Thus, for example for a body portion of a uniform material (say just water) but of a non-uniform shape (say rectangular) an acquisition at a projection through one direction of the body portion (say down the long axis of the rectangle) passes through more water than an acquisition at a projection that is orthogonal (down the short axis of the rectangle). The top layer of the dual layer X-ray detector that interacts with X-rays first is more sensitive to low energy X-rays and the bottom layer that interacts with X-rays that have passed though the top layer is more sensitive to high energy X-rays.

In the above situation, X-rays that pass through the long axis of the rectangle become "hardened" because the low energy X-rays are more likely to be absorbed than the high energy X-rays. Thus the X-ray beam hitting the detector has become beam hardened in that the X-ray spectrum has relatively more high energy X-rays than low energy X-rays (with respect to say a uniform shaped body portion). The signal in the bottom layer of the detector with respect to the top layer of the detector is then relatively increased.

In the above situation, the opposite occurs with respect to X-rays that pass through the short axis of the rectangle. The X-ray beam hitting the detector has not become beam hardened rather has been "softened" with respect to the beam that passed through the long axis of the rectangle. The signal in the bottom layer of the detector with respect to the top layer of the detector is then relatively decreased.

The optimum signal to noise cannot then be obtained in both cases, where ideally the signal strength in both layers of the dual layer detector should be similar.

However, in the new system the X-ray tube voltage is varied. Thus in the above situation, the X-ray tube voltage is lower for the projection down the long axis of the rectangle with respect to the short axis of the rectangle. This is because the X-ray spectra changes with X-ray tube voltage, with the peak in voltage moving to lower energies as the X-ray tube voltage is reduced. The overall X-ray emission reduces as the X-ray tube voltage decreases, however this can be offset by varying the X-ray tube current that maintain the X-ray spectra shape for an X-ray tube voltage but varies the overall X-ray emission.

Therefore, the new system in varying the X-ray tube voltage as required enables the signal to noise ratio of the detector to be at or near an optimum level for each projection of an overall scan. Then, when it comes to material decomposition from the spectral energy data (from both layers of the detector) the detector data can be used with the X-ray tube voltage, that enables the X-ray spectra for each acquisition and its associated projection to be known, to provide for improved material decomposition with an overall reduction in X-ray dosage of the patient because the detector is operating at or near an optimum level for each acquisition and its associated projection.

In an example, the information on the body portion for each projection of the different projections angles comprises a thickness of the body portion for each projection of the different projections angles.

In an example, the information on the body portion for each projection of the different projections angles comprises an X-ray attenuation of the body portion for each projection of the different projections angles.

In an example, the determination of the material decomposition imagery of the body portion comprises utilization of X-ray spectra associated with each determined X-ray tube voltage for each acquisition of the implemented overall scan.

In an example not forming part of the invention, the processing unit is configured to control the spectral X-ray CT imaging unit to carry out a single 2D scanogram of the body portion, and the processing unit is configured to receive data from the dual layer X-ray detector for the single 2D scanogram of the body portion. The processing unit is configured to determine the information on the body portion for each projection of the different projections angles on the basis of the data from the dual layer X-ray detector for the single 2D scanogram of the body portion.

In an example, the processing unit is configured to control the spectral X-ray CT imaging unit to carry out two 2D scanograms of the body portion, and the processing unit is configured to receive data from the dual layer X-ray detector for the two 2D scanograms of the body portion. The processing unit is configured to determine the information on the body portion for each projection of the different projections angles on the basis of the data from the dual layer X-ray detector for the two 2D scanograms of the body portion.

In an example, the two 2D scanograms of the body portion are two orthogonal 2D scanograms of the body portion.

In an example, the system comprises additionally at least one visible or infrared camera. The processing unit is configured to control the at least one visible and/or infrared camera to acquire visible and/or IR image data of the body portion, and the processing unit is configured to receive the visible and/or IR image data of the body portion. The processing unit is configured to determine the information on the body portion for each projection of the different projections angles additionally on the basis of the visible and/or IR image data of the body portion.

Thus, a camera system can be utilized from which the shape and thus thickness through the patient can be determined for different projections at different angles around the patient. The camera system can be a dual camera stereo system enabling depth data to be determined, or a LIDAR based depth determination system for example. This can be combined with X-ray data acquired via a single 2D scanogram or double scanograms to provide information of thicknesses and attenuation profiles of the body portion through different projections enabling a better determination of X-ray voltage to be made.

**In** an example, the determination of the voltage for the X-ray tube for each acquisition of the plurality of acquisitions comprises a comparison of the corresponding information on the body portion for the projection associated with that acquisition with reference data of different information of body portions versus different X-ray tube voltages.

Thus for example, a database can have combinations of different thicknesses against different tube voltages that provide for optimum detector performance, enabling the required X-ray tube voltage to be determined based on a thickness per projection.

Thus for example, a database can have combinations of different attenuations against different tube voltages that provide for optimum detector performance, enabling the required X-ray tube voltage to be determined based on an attenuation per projection.

Thus for example, a database can have combinations of different thickness and attenuation profiles against different tube voltages that provide for optimum detector performance, enabling the required X-ray tube voltage to be determined based on a thickness and attenuation profile per projection.

In an example, the determination of the voltage for the X-ray tube for each acquisition of the plurality of acquisitions comprises a determination of an X-ray tube voltage that is determined to result in substantially equivalent signal strength in both the top detector layer and bottom detector layer of the dual layer X-ray detector for the corresponding information on the body portion for the projection associated with that acquisition.

In an example, the determination of the voltage for the X-ray tube for each acquisition of the plurality of acquisitions comprises a determination of an X-ray tube voltage that is determined to result in a maximum signal to noise ratio for the data from the dual layer X-ray detector for the corresponding information on the body portion for the projection associated with that acquisition.

In an example, the processing unit is configured to determine a current for the X-ray tube for each acquisition of the plurality of acquisitions. The determination for each acquisition comprises utilization of the corresponding information on the body portion for the projection associated with that acquisition.

Thus, for a body portion of uniform material composition the X-ray tube voltage for a thicker projection will be less than the X-ray tube voltage for a thinner projection, in that these two situations can result in similar signal strenth in both layers of the detector. However, to account for a reduction in overall X-ray emission as the X-ray tube voltage decreases the X-ray tube current can be increased if required to increase the X-ray emission without changing the X-ray spectrum.

Also, the X-ray tube current can be altered in a manner similar to the traditional dose modulation technique in order to account for globally different shapes and thicknesses of parts of the patient in order to maintain a uniform dosage via modulation of the X-ray tube current.

In an example, the determination of the current for the X-ray tube for each acquisition of the plurality of acquisitions comprises a determination of an X-ray tube current that is determined to result in a substantially same X-ray dose received by the body portion for each acquisition.

In a second aspect, there is provided a spectral X-ray CT imaging method according to claim 12.

According to another aspect, there is provided a computer program element controlling one or more of the systems as previously described which, if the computer program element is executed by a processor, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic example of a spectral X-ray CT imaging system;
Fig. 2 shows a spectral X-ray CT imaging method;
Fig. 3 shows a detailed workflow of material decomposition undertaken by the system of fig. 1 and the method of Fig. 2;
Fig. 4 shows exemplar signal to noise ratios in Mono-70 images;
Fig. 5 shows exemplar X-ray tube voltages and signal to noise gain;
Fig. 6 shows exemplar signal to noise ratios in Mono-70 images and shows exemplar X-ray tube voltages and signal to noise gain; and
Fig. 7 shows a schematic representation of a dual layer X-ray detector.

### DETAILED DESCRIPTION OF EMBODIMENTS

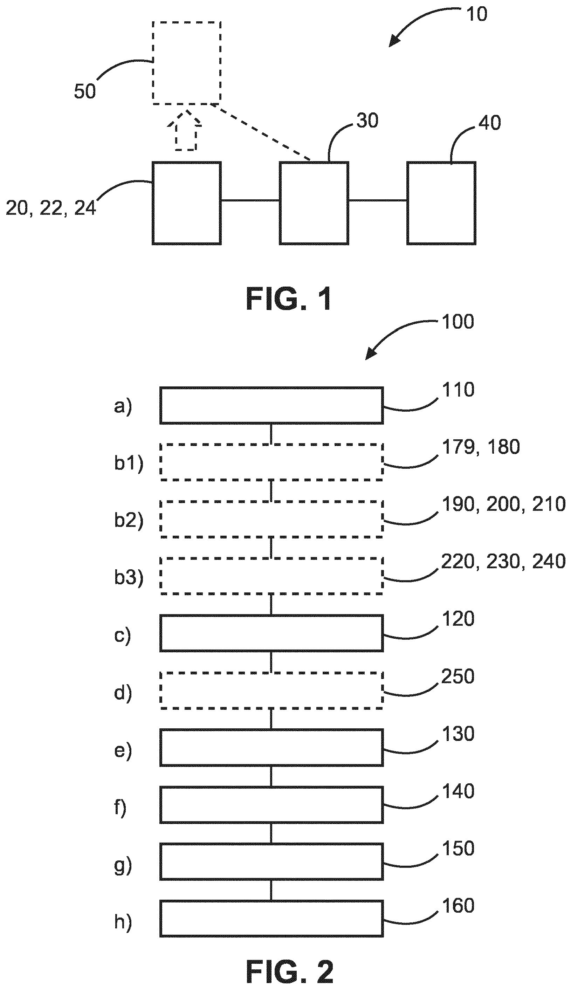

Fig. 1 shows an example of a spectral X-ray CT imaging system 10. The system 10 comprises a spectral X-ray CT imaging unit 20, a processing unit 30, and an output unit 40. The spectral X-ray CT imaging unit comprises an X-ray tube 22 and a dual layer X-ray detector 24. A body portion of a subject to be examined can be located between the X-ray tube and the dual layer X-ray detector. The spectral X-ray CT imaging unit is configured to acquire an overall scan of the body portion comprising a plurality of acquisitions at different projections angles. This can be through rotating the X-ray tube and detector around the body portion - thus each acquisition is at a different projection angle through the body portion. The processing unit is configured to utilize information on the body portion for each projection of the different projections angles. The processing unit is configured to determine a voltage for the X-ray tube for each acquisition of the plurality of acquisitions. The determination of the voltage for the X-ray tube for each acquisition of the plurality of acquisitions comprises utilization of the corresponding information on the body portion for the projection associated with that acquisition. The processing unit is configured to control the spectral X-ray CT imaging unit to carry out an implemented overall scan of the body portion. The control comprises controlling the X-ray tube to operate at the determined X-ray tube voltage for each acquisition of the plurality of acquisitions at the different projections angles. The processing unit is configured to receive data from the dual layer X-ray detector for each acquisition of the implemented overall scan. The processing unit is configured to implement a machine learning algorithm to determine material decomposition imagery of the body portion. The determination of the material decomposition imagery of the body portion comprises utilization of the data from the dual layer X-ray detector for each acquisition of the implemented overall scan and the determined X-ray tube voltage for each acquisition of the implemented overall scan. The output unit is configured to output the material decomposition imagery of the body portion.

In an example, the same X-ray tube voltage is determined for two acquisitions for which the information on the body portion for the projections associated with the two acquisitions is the same.

Thus, two different X-ray tube voltages are determined for two acquisitions for which the information on the body portion for the projections associated with the two acquisitions is different.

In an example, the machine learning algorithm is at least one neural network.

In an example, the information on the body portion for each projection of the different projections is derived from information of the shape of the subject.

Alternatively or additionally the information on the body portion for each projection of the different projections is derived from information of the thickness of the subject and/or information of the total X-ray absorption or X-ray attenuation of the subject and/or information on X-ray absorption profiles or X-ray attenuation profiles of the subject.

According to an example, the information on the body portion for each projection of the different projections angles comprises a thickness of the body portion for each projection of the different projections angles.

According to an example, the information on the body portion for each projection of the different projections angles comprises an X-ray attenuation of the body portion for each projection of the different projections angles.

According to an example, the determination of the material decomposition imagery of the body portion comprises utilization of X-ray spectra associated with each determined X-ray tube voltage for each acquisition of the implemented overall scan.

According to an example, the processing unit is configured to control the spectral X-ray CT imaging unit to carry out a single 2D scanogram of the body portion. The processing unit is configured to receive data from the dual layer X-ray detector for the single 2D scanogram of the body portion. The processing unit is configured to determine the information on the body portion for each projection of the different projections angles on the basis of the data from the dual layer X-ray detector for the single 2D scanogram of the body portion.

According to an example, the processing unit is configured to control the spectral X-ray CT imaging unit to carry out two 2D scanograms of the body portion. The processing unit is configured to receive data from the dual layer X-ray detector for the two 2D scanograms of the body portion. The processing unit is configured to determine the information on the body portion for each projection of the different projections angles on the basis of the data from the dual layer X-ray detector for the two 2D scanograms of the body portion.

According to an example, the two 2D scanograms of the body portion are two orthogonal 2D scanograms of the body portion.

According to an example, the system comprises at least one visible or infrared camera 50. The processing unit is configured to control the at least one visible and/or infrared camera to acquire visible and/or IR image data of the body portion. The processing unit is configured to receive the visible and/or IR image data of the body portion. The processing unit is configured to determine the information on the body portion for each projection of the different projections angles on the basis of the visible and/or IR image data of the body portion.

According to an example, the determination of the voltage for the X-ray tube for each acquisition of the plurality of acquisitions comprises a comparison of the corresponding information on the body portion for the projection associated with that acquisition with reference data of different information of body portions versus different X-ray tube voltages.

According to an example, the determination of the voltage for the X-ray tube for each acquisition of the plurality of acquisitions comprises a determination of an X-ray tube voltage that is determined to result in substantially equivalent signal strength in both the top detector layer and bottom detector layer of the dual layer X-ray detector for the corresponding information on the body portion for the projection associated with that acquisition.

In an example, calculations can be carried out to determine the X-ray voltage that provides for an optimum detector operation based on thickness, or attenuation or a combination of thickness and attenuation per projection.

In an example, comparison with a database of values can be done to provide for an X-ray voltage that is expected to result in equivalent strength in both layers of the detector for each projection.

According to an example, the determination of the voltage for the X-ray tube for each acquisition of the plurality of acquisitions comprises a determination of an X-ray tube voltage that is determined to result in a maximum signal to noise ratio for the data from the dual layer X-ray detector for the corresponding information on the body portion for the projection associated with that acquisition.

According to an example, the processing unit is configured to determine a current for the X-ray tube for each acquisition of the plurality of acquisitions. The determination of the current for the X-ray tube for each acquisition comprises utilization of the corresponding information on the body portion for the projection associated with that acquisition.

In an example, the same X-ray tube current is determined for two acquisitions for which the information on the body portion for the projections associated with the two acquisitions is the same.

Thus, two different X-ray tube currents are determined for two acquisitions for which the information on the body portion for the projections associated with the two acquisitions is different.

According to an example, the determination of the current for the X-ray tube for each acquisition of the plurality of acquisitions comprises a determination of an X-ray tube current that is determined to result in a substantially same X-ray dose received by the body portion for each acquisition.

Fig. 2 shows a spectral X-ray CT imaging method 100 in its basic steps. The method 100 comprises:
in a locating step 110, also referred to as step a), locating a body portion of a subject to be examined between an X-ray tube and a dual layer X-ray detector of a spectral X-ray CT imaging unit, and wherein the spectral X-ray CT imaging unit is configured to acquire an overall scan of the body portion comprising a plurality of acquisitions at different projections angles;
in a utilizing and determining step 120, also referred to as step c), utilizing by a processing unit information on the body portion for each projection of the different projections angles, and determining by the processing unit a voltage for the X-ray tube for each acquisition of the plurality of acquisitions, wherein the determining for each acquisition comprises utilizing the corresponding information on the body portion for the projection associated with that acquisition;
in a controlling step 130, also referred to as step e), controlling by the processing unit the spectral X-ray CT imaging unit to carry out an implemented overall scan of the body portion, wherein the control comprises controlling the X-ray tube to operate at the determined X-ray tube voltage for each acquisition of the plurality of acquisitions at the different projections angles;
in a receiving step 140, also referred to as step f), receiving by the processing data from the dual layer X-ray detector for each acquisition of the implemented overall scan;
in an implementing and determining step 150, also referred to as step g),
implementing (150) by the processing unit a machine learning algorithm and determining by the machine learning algorithm material decomposition imagery of the body portion, wherein the determining comprises utilizing the data from the dual layer X-ray detector for each acquisition of the implemented overall scan and the determined X-ray tube voltage for each acquisition of the implemented overall scan; and
in an outputting step 160, also referred to as step h), outputting by an output unit the material decomposition imagery of the body portion.

In an example, the same X-ray tube voltage is determined for two acquisitions for which the information on the body portion for the projections associated with the two acquisitions is the same.

Thus, two different X-ray tube voltages are determined for two acquisitions for which the information on the body portion for the projections associated with the two acquisitions is different.

In an example, the information on the body portion for each projection of the different projections angles comprises a thickness of the body portion for each projection of the different projections angles.

In an example, the information on the body portion for each projection of the different projections comprises an X-ray attenuation of the body portion for each projection of the different projections.

In an example, determining the material decomposition imagery of the body portion comprises utilizing X-ray spectra associated with each determined X-ray tube voltage for each acquisition of the implemented overall scan.

In an example, the method comprises b1) controlling 170 by the processing unit the spectral X-ray CT imaging unit to carry out a single 2D scanogram of the body portion, and receiving by the processing unit data from the dual layer X-ray detector for the single 2D scanogram of the body portion, and determining 180 by the processing unit the information on the body portion for each projection of the different projections angles on the basis of the data from the dual layer X-ray detector for the single 2D scanogram of the body portion.

In an example, the method comprises b2) controlling 190 by the processing unit the spectral X-ray CT imaging unit to carry out two 2D scanograms of the body portion, and receiving 200 by the processing unit data from the dual layer X-ray detector for the two 2D scanograms of the body portion, and determining 210 by the processing unit the information on the body portion for each projection of the different projections angles on the basis of the data from the dual layer X-ray detector for the two 2D scanograms of the body portion.

In an example, the two 2D scanograms of the body portion are two orthogonal 2D scanograms of the body portion.

In an example, the method comprises b3) controlling 220 by the processing unit at least one visible and/or infrared camera to acquire visible and/or IR image data of the body portion, and receiving 230 by the processing unit the visible and/or IR image data of the body portion, and determining 240 by the processing unit the information on the body portion for each projection of the different projections angles on the basis of the visible and/or IR image data of the body portion.

In an example, the determining the voltage for the X-ray tube for each acquisition of the plurality of acquisitions comprises comparing the corresponding information on the body portion for the projection associated with that acquisition with reference data of different information of body portions versus different X-ray tube voltages.

In an example, the determining the voltage for the X-ray tube for each acquisition of the plurality of acquisitions comprises determining an X-ray tube voltage that is determined to result in substantially equivalent signal strength in both the top detector layer and bottom detector layer of the dual layer X-ray detector for the corresponding information on the body portion for the projection associated with that acquisition.

In an example, the determining the voltage for the X-ray tube for each acquisition of the plurality of acquisitions comprises determining an X-ray tube voltage that is determined to result in a maximum signal to noise ratio for the data from the dual layer X-ray detector for the corresponding information on the body portion for the projection associated with that acquisition.

In an example, the method comprises d) determining (250) by the processing unit a current for the X-ray tube for each acquisition of the plurality of acquisitions, wherein the determining for each acquisition comprises utilizing the corresponding information on the body portion for the projection associated with that acquisition.

In an example, the determining the current for the X-ray tube for each acquisition of the plurality of acquisitions comprises determining an X-ray tube current that is determined to result in a substantially same X-ray dose received by the body portion for each acquisition.

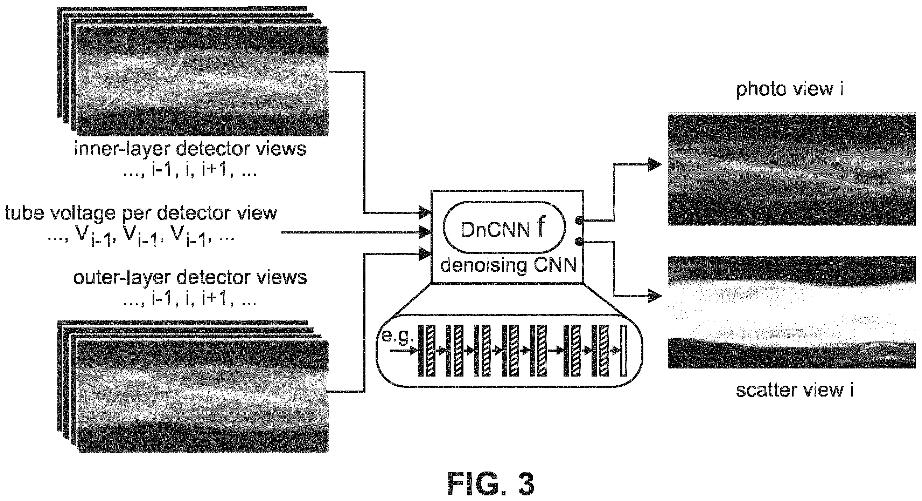

The spectral X-ray CT imaging system and method are further explained in specific detail, where reference is made to Figs. 3-7.

As discussed above with respect to Figs. 1-2 the new approach reduces the negative effects of the limitations of existing material decomposition through the usage of projection dependent tube voltages which can be used to achieve balanced signal per energy channel on the projections. Material decomposition into photo and scatter projections can then be achieved using a kVp informed neural network for noise robust material decomposition.

In order to achieve optimal spectral quality and separation in spectral CT imaging an optimal photon level per energy channel is required on each measured CT projection, and the new approach described here facilitates this. This optimal photon number per energy channel is achieved and utilized in combination with a neural network for kV selective material decomposition.

In essence, the new approach enables an estimation/determination to be made of the X-ray tube voltage per projection view. This leads to, depending on the object under examination, the ability to achieve an optimal balance of the photon flux per energy channel in the dual layer detector, and enables material decomposition through the use of a neural network for AI based material decomposition which receives information on the used tube voltage per projection. This approach is unique because it allows for varying (optimized) spectra within one scan with an AI guided correction method implemented in the material decomposition. This approach allows for:
Patient specific scan optimization because the dynamic kVp- modulation follows the patient shape.

Clinical application specific scan optimization because the optimization criteria may be selected based on clinical needs.

The new approach in detail can be described as follows:
A dual energy detection detector is utilized, with a first part or layer that detects X-rays over a first energy range and a second part that detects X-rays over a second energy range - first and second parts stacked on top of each other. Here the first part interacts with X-rays first and absorbs X-rays over a low energy range - the first energy range, and the second part that is below the first part absorbs X-rays over a high energy range - the second energy range.

An X-ray tube at a high voltage to produce a spectrum of X-rays over the whole energy range. Normally when taking many scans to generate an overall CT scan the X-ray tube voltage is held constant. However now the X-ray tube voltage is varied as required. Changing the x-ray tube voltage leads to a change in the X-ray spectrum, with an increase in X-ray tube voltage leading to the peak in emission moving to higher energies. The current of the X-ray tube can be varied to increase the X-ray emission for a constant X-ray spectrum.

Prior information on the different tube voltages for different projections through a person that will produce the "optimum spectrum of X-rays" for each projection. Prior knowledge of the patient shape/thicknesses can be generated that enables the estimation of optimal tube voltages per projection. Here, ideally what is wanted is there to be the same number of photons per scan being detected in the first part and being detected in the second part of the detector.

For a person about to undertake an overall CT scan comprising many scans at different angles/projections (so different thicknesses) this prior knowledge can be generated by doing one of the following (alone or indeed in combination):
Use a camera to assess the geometry of the person. Here the thickness of the patient can be estimated.

Carry out one low dose 2D scanogram. Here total absorption can be determined.

Carry out two orthogonal low dose 2D scanograms. Here thickness and attenuation profiled can be determined.

Carry out a multi-scan low dose 3D scanogram. Here thickness and attenuation profiled can be determined.

It is to be noted that even rough estimates of size and/or attenuation can enable an X-ray tube voltage to be utilized per projection that may not be the absolute optimum, but will be optimised with respect to an x-ray tube voltage associated with a fixed X-ray tube voltage for all projections.

A full CT scan is then carried out, where for each projection it is known from 3 and 4 above what X-ray tube voltage is optimum for each projection and the X-ray tube voltage varies accordingly with the projections. Here, the X-ray tube current can be changed as well to account for a reducing X-ray yield due to a decrease in X-ray tube voltage that can be compensated by increasing the X-ray tube current as required, and modulation of the X-ray tube current to account for different body part sizes to maintain the X-ray dose across the body.

The data for each projection (scan) of the overall scan from both detector parts is provided to a neural network along with the tube voltage (that enables the associated x-ray spectra to be calculated or retried from a database) used for each projection (scan) and the NN determines the material decomposition data (such as photoelectric scattering and Compton scattering data sets). However, now the material decomposition is optimized because as every scan of the overall scan was at an optimum tube voltage. This also means that the overall x-ray dose to the patient can be minimised.

Fig. 3 shows a representation of the material decomposition. On the left are shown the data inputs per projection for the two detector parts or layers, obtained at different X-ray tube voltages. These data, along with the X-ray tube voltage used for each projection, that enables the corresponding X-ray spectrum to be utilized, are provided to the neural network. This then carries out material decomposition, for example outputting Photoelectric scattering and Compton scattering image data sets.

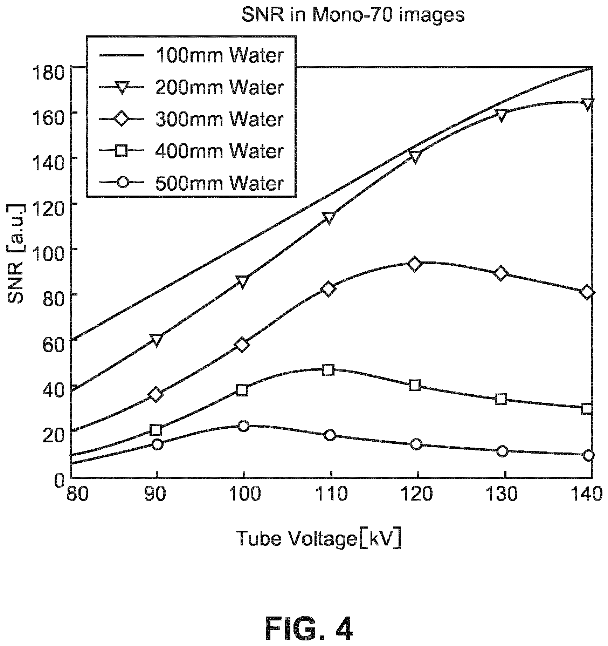

The determination of the required X-ray tube voltage is explained with reference to Figs. 4-5.

It first must be highlighted that the new approach leads to what appears to be a contra intuitive operational methodology. This is because at a basic level if an object gets thicker, one should decrease the tube voltage to compensate the stronger beam hardening to get a better balance of the flux in the two detector layers (and vice versa). This may appear rather surprising, until beam hardening as discussed herein is taken into consideration - and of course one can increase X-ray tube current as the X-ray voltage is reduced to maintain a required X-ray flux and signal level.

Fig. 4 shows the signal to noise in calculated image for a spectral CT scan. Here a calculated Mono-70 image from a spectral CT scan, represented the expected result from a monochromatic scan with 70keV photons.

The Mono-70 image representing the expected result from a monochromatic scan at 70keV enables a signal to noise ratio SNR to be calculated for objects of different thicknesses. The SNR relates to the signals in both parts or layers of the dual energy detector and will tend to a maximum as the signals in both parts or layers tend toward the same magnitude.

Fig. 4 then shows the SNR for such "Mono-70" images but calculated for different X-ray tube voltages and for different object thicknesses (the objects here are made from water). In Fig. 4 the detector design has been optimized for a 300mm object size and a tube voltage of 120kV. The generation of mono-70 images is then taken as a benchmark imaging task for which it is wanted to optimize the signal-to-noise ratio (for example called SNR-70). Then this quantity (SNR-70) can be calculated for different tube voltages. However, changing the tube voltage will change the X-Ray patient dose. To compensate for this effect, the tube current can be adapted such that the dose (measured in KERMA) will stay constant. Figure 4 shows these SNR-70 values for some object diameters over a range of X-ray tube voltages. It can clearly be seen that the optimal tube voltage depends on the object diameter and that the optimal voltage decreases with larger object diameter. For example the optimum for 300mm water is at about 120 kV.

The curves in Fig. 4 can be understood as follows. The upper detector layer is more sensitive to low energy X-Rays (and vice versa). With increasing patient thickness there is more attenuation in the patient and therefore more "beam hardening" with respect to a beam exiting the patient that then interacts with the detector. This is because low energy photons have a higher absorption likelihood and the beam gets harder (mean energy goes up). This leads to relatively more signal in the lower detector layer (the detector layer that detects higher energy X-rays). This "imbalance" leads to a decreased SNR in Mono70. To recall, for a 300mm thick object curve it is seen that 120kVp is the optimal voltage. However, this optimum changes with patient thickness as indicated with the other curves, moving to lower X-ray tube voltages as the thickness increases and to higher X-ray tube voltages as the thickness decreases.

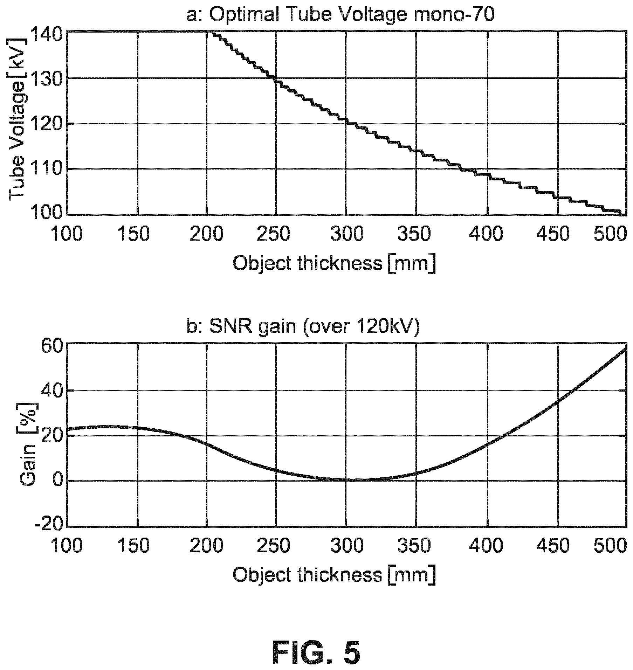

The best tube voltage can then be estimated over the patient diameter (see Fig. 5a) and the gain of the SNR-70 can be estimated in comparison to the optimal tube voltage with the current standard of 120kV (see Fig. 5b).

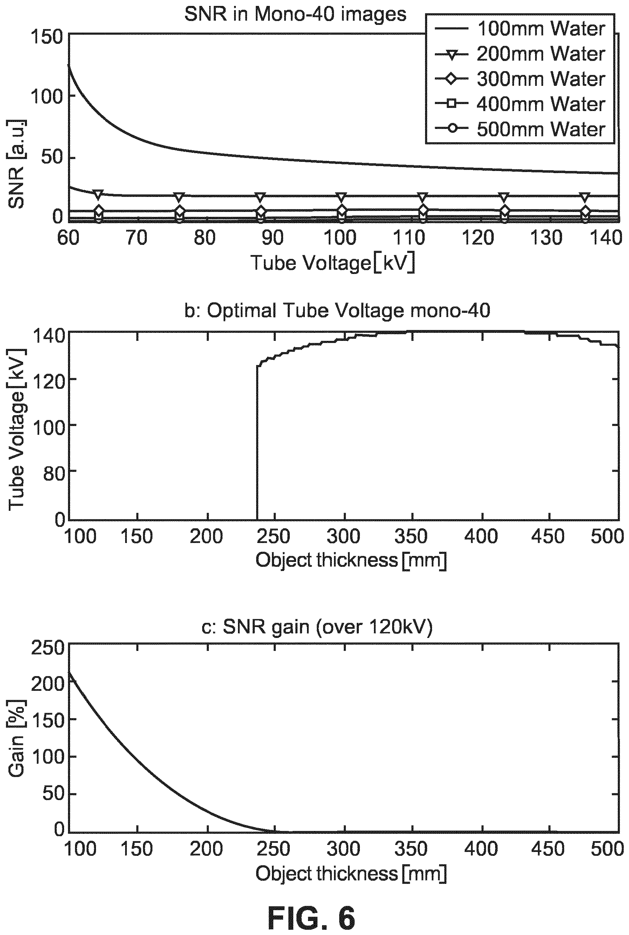

This result indicates that there is a dependency on the optimization criteria (SNR-70) for our imaging task. Although this image is close to a conventional image made with 120kVp, the clinically relevant optimum might be somewhere else. This indicates that for a conventional imaging mode, the data presented with respect to MON0-70 and SNR-70 could be optimal. However, if the imaging task is to optimize the contrast-to-noise ratio for a contrast agent relative to tissue, one may want to optimize for lower energy mono-E images (e.g. mono-40). Fig. 6 shows the related results for Mono-40.

Thus, depending on the image task knowledge of the patient size (and attenuation characteristics) determined from a pre-scanogram or via camera imagery enables the optimum X-ray tube voltage per projection to be determined, and this information along with the detector spectral data for each projection can be processed by a neural network to provide optimized material decomposition.

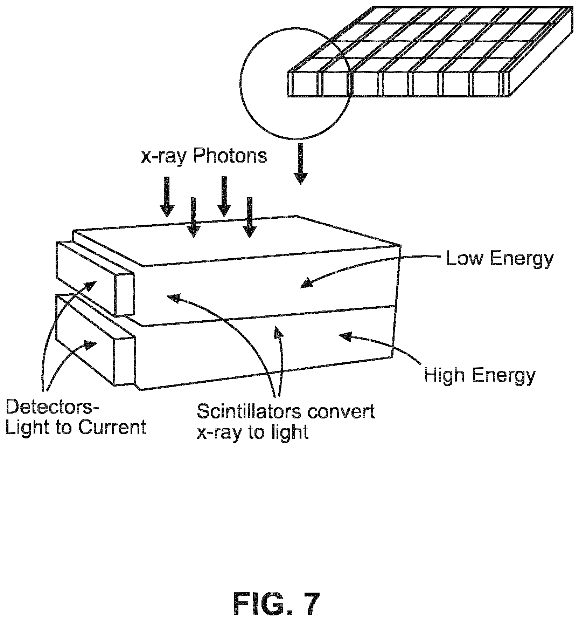

Reference has been made above to a dual layer detector. Fig. 7 shows an exemplary dual layer detector array, which is a stack of two scintillators that are used to obtain spectral information by different effective spectral sensitivities of the layers, with one pixel of that array shown in an expanded view. A detector pixel is made from two scintillators stacked one on top of the other, with X-rays being incident from the top. Low energy X-rays are absorbed in the top scintillators, with absorption leading to the emission of longer wavelength radiation that is detected by a photodiode that is positioned on the lateral side of that scintillator. The bottom scintillator absorbs high energy X-rays and again re-emitted longer wavelength radiation is detected by a second photodiode associated with that scintillator.

The following relates to how the neural network for material decomposition can be informed with the available scalar prior information (kVp setting for each projection view).

There are two general approaches to do this:
One can have multiple networks that are dedicatedly trained for a certain (range of) kVp setting(s). This can be achieved, e.g., by subdividing the whole range of possible kVp's into smaller (potentially overlapping) sub-intervals, and training a net for each interval (e.g., by sampling from the interval during training). Alternatively, one may select a discrete subset from the range of possible kVp settings, and train a network for each setting. In both cases, in inference, for a given (range of) projection view(s) a corresponding network needs to be selected. This can be done with a look up table (LUT) that contains the information about which network(s) were trained for which kVp setting(s). Alternatively, if the networks were each trained for a single kVP setting, one may use the nearest neighbor of these kVp values to the given kVp setting. In general, if there are more than one network for a given kVp setting, one may compute an (weighted) average over the outputs of these networks; but any other method for combining the outputs of the different networks could also be used (one may even train a machine learning algorithm for this). It would also be possible to combine the outputs of multiple networks in a spatially varying manner.

In other embodiments, there may be a (number of) network(s) that have a built-in mechanism for adapting to a given kVp setting. There are different ways to achieve this:
One way is to add an additional input channel to the network, which receives the kVP setting as prior-information input during both training and inference. Since the network input to the different input channels may be 2D or 3D, the scalar information (kVp setting) may be broadcasted to a map which is of the same size and dimensionality as for the other input channels of the network. During training, the network can then learn how to use the available prior information to better perform on the task it is trained for (material decomposition). This learned mechanism will then help the network to better perform also during inference. This may also yield a more robust network that can better generalize to kVp settings not seen during training.

In other embodiments, one may use a certain type of activation function in the network, such as soft-shrinkage activation functions, or add additional components to the network, such as attention gates, to inform the network with the scalar prior information. Another possibility is to use so-called loss-conditional training of networks. In this technique, the network is trained with a distribution of loss functions for different kVp settings. In inference the model can then be conditioned by the given kVp setting to generate an output corresponding to a particular loss from this distribution.

Yet another method for informing the network with the kVp setting is to scale the internal feature maps of the network with the scalar prior information. This is typically performed on the output of the convolutional layers of the network, before passing the feature maps on to the subsequent non-linearity/activation function, but any other type of scaling (a subset of) the internal features of the network may also work.

All of the above-described techniques can also be used with multiple sources of scalar prior information, e.g., if not only the kVp setting but also the intensity of the primary beam is provided.

Last but not least, it is to be noted with respect to the "input interface" of a network if the prior information is used "within" the network (e.g., when using attention gates), the input interface of the network can be considered to receive the scalar information just as it would if the prior information was fed to additional input channels to the neural net.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this patent.

However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A spectral X-ray CT imaging system (10), comprising:
- a spectral X-ray CT imaging unit (20);
- a processing unit (30); and
- an output unit (40);
wherein the spectral X-ray CT imaging unit comprises an X-ray tube (22) and a dual layer X-ray detector (24), and wherein a body portion of a subject to be examined can be located between the X-ray tube and the dual layer X-ray detector;
wherein the processing unit is configured to acquire two 2D scanograms of the body portion at different projection angles or a 3D scanogram of the body portion;
wherein the processing unit is configured to utilize thickness and attenuation profiles of the body portion through different projections from the two 2D scanograms or from the 3D scanogram, wherein the profiles enable determination of a voltage for the X-ray tube for each acquisition of a plurality of acquisitions of an implemented overall scan;
wherein the processing unit is configured to control the spectral X-ray CT imaging unit to carry out the implemented overall scan of the body portion, wherein the control comprises controlling the X-ray tube to operate at the determined X-ray tube voltage for each acquisition of the plurality of acquisitions at the different projection angles of the implemented overall scan;
wherein the processing unit is configured to receive data from the dual layer X-ray detector for each acquisition of the implemented overall scan;
wherein the processing unit is configured to implement a machine learning algorithm to determine material decomposition imagery of the body portion, wherein the determination comprises utilization of the data from the dual layer X-ray detector for each acquisition of the implemented overall scan and the determined X-ray tube voltage for each acquisition of the implemented overall scan; and
wherein the output unit is configured to output the material decomposition imagery of the body portion.

2. System according to claim 1, wherein the patient size and attenuation characteristics information on the body portion for each projection of the different projection angles comprises a thickness of the body portion for each projection of the different projection angles.

3. System according to any of claims 1-2, wherein the patient size and attenuation characteristics information on the body portion for each projection of the different projection angles comprises an X-ray attenuation of the body portion for each projection of the different projection angles.

4. System according to any of claims 1-2, wherein determination of the material decomposition imagery of the body portion comprises utilization of X-ray spectra associated with each determined X-ray tube voltage for each acquisition of the implemented overall scan.

5. System according to claim 1, wherein the two 2D scanograms of the body portion are two orthogonal 2D scanograms of the body portion.

6. System according to any of claims 1-2, wherein the system comprises at least one visible or infrared camera (50), wherein the processing unit is configured to control the at least one visible and/or infrared camera to acquire visible and/or IR image data of the body portion, wherein the processing unit is configured to receive the visible and/or IR image data of the body portion, and wherein the processing unit is additionally configured to determine the thicknesses and attenuation profiles of the body portion for each projection of the different projection angles on the basis of the visible and/or IR image data of the body portion.

7. System according to any of claims 1-2, wherein the determination of the voltage for the X-ray tube for each acquisition of the plurality of acquisitions comprises a comparison of the corresponding patient size and attenuation characteristics information on the body portion for the projection associated with that acquisition with reference data of different information of body portions versus different X-ray tube voltages.

8. System according to any of claims 1-2, wherein the determination of the voltage for the X-ray tube for each acquisition of the plurality of acquisitions comprises a determination of an X-ray tube voltage that is determined to result in substantially equivalent signal strength in both the top detector layer and bottom detector layer of the dual layer X-ray detector for the corresponding patient size and attenuation characteristics information on the body portion for the projection associated with that acquisition.

9. System according to any of claims 1-2, wherein the determination of the voltage for the X-ray tube for each acquisition of the plurality of acquisitions comprises a determination of an X-ray tube voltage that is determined to result in a maximum signal to noise ratio for the data from the dual layer X-ray detector for the corresponding patient size and attenuation characteristics information on the body portion for the projection associated with that acquisition.

10. System according to any of claims 1-2, wherein the processing unit is configured to determine a current for the X-ray tube for each acquisition of the plurality of acquisitions, wherein the determination for each acquisition comprises utilization of the corresponding patient size and attenuation characteristics information on the body portion for the projection associated with that acquisition.

11. System according to claim 12, wherein the determination of the current for the X-ray tube for each acquisition of the plurality of acquisitions comprises a determination of an X-ray tube current that is determined to result in a substantially same X-ray dose received by the body portion for each acquisition.

12. A spectral X-ray CT imaging method (100), comprising:
a) locating (110) a body portion of a subject to be examined between an X-ray tube and a dual layer X-ray detector of a spectral X-ray CT imaging unit, and wherein the spectral X-ray CT imaging unit is configured to acquire two 2D scanograms of the body portion comprising a plurality of acquisitions at different projection angles or a 3D scanogram of the body portion;
c) by utilizing, by a processing unit, thickness and attenuation profiles of the body portion through different projections from the two 2D scanograms or from the 3D scanogram, wherein the profiles enable determining (120) by the processing unit a voltage for the X-ray tube for each acquisition of a plurality of acquisitions of an implemented overall scan,
e) controlling (130) by the processing unit the spectral X-ray CT imaging unit to carry out an implemented overall scan of the body portion, wherein the control comprises controlling the X-ray tube to operate at the determined X-ray tube voltage for each acquisition of the plurality of acquisitions at the different projection angles of the implemented overall scan;
f) receiving (140) by the processing unit data from the dual layer X-ray detector for each acquisition of the implemented overall scan;
g) implementing by the processing unit a machine learning algorithm and determining (150) by the machine learning algorithm material decomposition imagery of the body portion, wherein the determining comprises utilizing the data from the dual layer X-ray detector for each acquisition of the implemented overall scan and the determined X-ray tube voltage for each acquisition of the implemented overall scan; and
h) outputting (160) by an output unit the material decomposition imagery of the body portion.

13. A computer program element for controlling a system according to any of claims 1-2, which when executed by a processor is configured to carry out the method of claim 12.

## Patentansprüche

1. Spektrales Röntgen-CT-Bildgebungssystem (10), umfassend:
- eine spektrale Röntgen-CT-Bildgebungseinheit (20);
- eine Verarbeitungseinheit (30); und
- eine Ausgabeeinheit (40);
wobei die spektrale Röntgen-CT-Bildgebungseinheit eine Röntgenröhre (22) und einen Doppelschicht-Röntgendetektor (24) umfasst und wobei sich ein Körperabschnitt einer zu untersuchenden Person zwischen der Röntgenröhre und dem Doppelschicht-Röntgendetektor befinden kann;
wobei die Verarbeitungseinheit so konfiguriert ist, dass sie zwei 2D-Scanogramme des Körperabschnitts unter verschiedenen Projektionswinkeln oder ein 3D-Scanogramm des Körperabschnitts aufnimmt;
wobei die Verarbeitungseinheit so konfiguriert ist, dass sie Dicken- und Dämpfungsprofile des Körperabschnitts durch verschiedene Projektionen aus den zwei 2D-Scanogrammen oder aus dem 3D-Scanogramm nutzt, wobei die Profile eine Bestimmung einer Spannung für die Röntgenröhre für jede Aufnahme einer Vielzahl von Aufnahmen eines implementierten Gesamtscans ermöglichen;
wobei die Verarbeitungseinheit so konfiguriert ist, dass sie die spektrale Röntgen-CT-Bildgebungseinheit steuert, um den implementierten Gesamtscan des Körperabschnitts auszuführen, wobei die Steuerung Steuern der Röntgenröhre umfasst, um sie für jede Aufnahme der Vielzahl von Aufnahmen bei den verschiedenen Projektionswinkeln des implementierten Gesamtscans mit der bestimmten Röntgenröhrenspannung zu betreiben;
wobei die Verarbeitungseinheit so konfiguriert ist, dass sie für jede Aufnahme des implementierten Gesamtscans Daten vom Doppelschicht-Röntgendetektor empfängt;
wobei die Verarbeitungseinheit so konfiguriert ist, dass sie einen Algorithmus für maschinelles Lernen implementiert, um Materialzerlegungsbilder des Körperabschnitts zu bestimmen, wobei die Bestimmung Nutzung der Daten des Doppelschicht-Röntgendetektors für jede Aufnahme des implementierten Gesamtscans und die für jede Aufnahme des implementierten Gesamtscans bestimmte Röntgenröhrenspannung umfasst; und
wobei die Ausgabeeinheit so konfiguriert ist, dass sie die Materialzerlegungsbilder des Körperabschnitts ausgibt.

2. System nach Anspruch 1, wobei die Informationen über Patientengröße und Dämpfungseigenschaften des Körperabschnitts für jede Projektion der verschiedenen Projektionswinkel eine Dicke des Körperabschnitts für jede Projektion der verschiedenen Projektionswinkel umfassen.

3. System nach einem der Ansprüche 1-2, wobei die Informationen über Patientengröße und Dämpfungseigenschaften des Körperabschnitts für jede Projektion der verschiedenen Projektionswinkel eine Röntgendämpfung des Körperabschnitts für jede Projektion der verschiedenen Projektionswinkel umfassen.

4. System nach einem der Ansprüche 1-2, wobei Bestimmung der Materialzerlegungsbilder des Körperabschnitts Nutzung von Röntgenspektren umfasst, die mit jeder bestimmten Röntgenröhrenspannung für jede Aufnahme des implementierten Gesamtscans verbunden sind.

5. System nach Anspruch 1, wobei die zwei 2D-Scanogramme des Körperabschnittszwei orthogonale 2D-Scanogramme des Körperabschnitts sind.

6. System nach einem der Ansprüche 1-2, wobei das System mindestens eine sichtbare oder Infrarotkamera (50) umfasst, wobei die Verarbeitungseinheit zur Steuerung der mindestens einen sichtbaren und/oder Infrarotkamera zum Aufnahmen von sichtbarem Licht und/oder IR-Bilddaten des Körperabschnitts konfiguriert ist, wobei die Verarbeitungseinheit so konfiguriert ist, dass sie die sichtbaren und/oder IR-Bilddaten des Körperabschnitts empfängt, wobei die Verarbeitungseinheit zusätzlich so konfiguriert ist, dass sie die Dicken- und Dämpfungsprofile des Körperabschnitts für jede Projektion der verschiedenen Projektionswinkel auf Basis der sichtbaren und/oder IR-Bilddaten des Körperbereichs bestimmt.

7. System nach einem der Ansprüche 1-2, wobei die Bestimmung der Spannung für die Röntgenröhre für jede Aufnahme der Vielzahl von Aufnahmen einen Vergleich der entsprechenden Informationen über Patientengröße und Dämpfungseigenschaften des Körperabschnitts für die mit der Aufnahme verbundene Projektion mit Referenzdaten über unterschiedliche Informationen über Körperabschnitte bei unterschiedlichen Röntgenröhrenspannungen umfasst.

8. System nach einem der Ansprüche 1-2, wobei die Bestimmung der Spannung für die Röntgenröhre für jede Aufnahme der Vielzahl von Aufnahmen eine Bestimmung einer Röntgenröhrenspannung umfasst, die so bestimmt wird, dass sie zu einer im Wesentlichen gleichwertigen Signalstärke sowohl in der oberen Detektorschicht als auch in der unteren Detektorschicht des Doppelschicht-Röntgendetektors für die entsprechenden Informationen über Patientengröße und Dämpfungseigenschaften des Körperabschnitts für die mit dieser Aufnahme verbundene Projektion führt.

9. System nach einem der Ansprüche 1-2, wobei die Bestimmung der Spannung für die Röntgenröhre für jede Aufnahme der Vielzahl von Aufnahmen eine Bestimmung einer Röntgenröhrenspannung umfasst, die so bestimmt wird, dass sie zu einem maximalen Signal-RauschVerhältnis für die Daten des Doppelschicht-Röntgendetektors für die entsprechenden Informationen über Patientengröße und Dämpfungseigenschaften des Körperabschnitts für die mit der Aufnahme verbundene Projektion führt.

10. System nach einem der Ansprüche 1-2, wobei die Verarbeitungseinheit so konfiguriert ist, dass sie für jede Aufnahme der Vielzahl von Aufnahmen einen Strom für die Röntgenröhre bestimmt, wobei die Bestimmung für jede Aufnahme Nutzung der entsprechenden Informationen über Patientengröße und Dämpfungseigenschaften des Körperabschnitts für die mit der Aufnahme verbundene Projektion umfasst.

11. System nach Anspruch 12, wobei die Bestimmung des Stroms für die Röntgenröhre für jede Aufnahme der Vielzahl von Aufnahmen eine Bestimmung eines Röntgenröhrenstroms umfasst, der so bestimmt wird, dass er zu einer im Wesentlichen gleichen Röntgendosis führt, die von dem Körperabschnitt für jede Aufnahme empfangen wird.

12. Spektrales Röntgen-CT-Bildgebungsverfahren (100), umfassend:
a) Positionieren (110) eines zu untersuchenden Körperabschnitts einer Person zwischen einer Röntgenröhre und einem Doppelschicht-Röntgendetektor einer spektralen Röntgen-CT-Bildgebungseinheit, wobei die spektrale Röntgen-CT-Bildgebungseinheit so konfiguriert ist, dass sie zwei 2D-Scanogramme des Körperabschnitts, umfassend eine Vielzahl von Aufnahmen unter verschiedenen Projektionswinkeln, oder ein 3D-Scanogramm des Körperabschnitts aufnimmt;
c) durch Nutzen, durch die Verarbeitungseinheit, von Dicken- und Dämpfungsprofilen des Körperabschnitts durch verschiedene Projektionen aus den beiden 2D-Scanogrammen oder aus dem 3D-Scanogramm, wobei die Profile Bestimmen (120) einer Spannung für die Röntgenröhre für jede Aufnahme einer Vielzahl von Aufnahmen eines implementierten Gesamtscans durch die Verarbeitungseinheit ermöglichen,
e) Steuern (130) durch die Verarbeitungseinheit der spektralen Röntgen-CT-Bildgebungseinheit, um einen implementierten Gesamtscan des Körperabschnitts auszuführen, wobei die Steuerung Steuern der Röntgenröhre umfasst, sodass diese bei der für jede Aufnahme der Vielzahl von Aufnahmen bei den verschiedenen Projektionswinkeln des implementierten Gesamtscans bestimmten Röntgenröhrenspannung arbeitet;
f) Empfangen (140) von Daten des Doppelschicht-Röntgendetektors durch die Verarbeitungseinheit für jede Aufnahme des implementierten Gesamtscans;
g) Implementieren, durch die Verarbeitungseinheit eines Algorithmus für maschinelles Lernen, und Bestimmen (150), durch den Algorithmus für maschinelles Lernen, von Materialzerlegungsbildern des Körperabschnitts, wobei das Bestimmen Nutzen der Daten des Doppelschicht-Röntgendetektors für jede Aufnahme des implementierten Gesamtscans und der für jede Aufnahme des implementierten Gesamtscans bestimmten Röntgenröhrenspannung umfasst; und
h) Ausgeben (160) durch eine Ausgabeeinheit der Materialzerlegungsbilder des Körperabschnitts.

13. Computerprogrammelement zum Steuern eines Systems nach einem der Ansprüche 1-2, das, wenn es durch einen Prozessor ausgeführt wird, dazu konfiguriert ist, das Verfahren nach Anspruch 12 auszuführen.

## Revendications

1. Système d'imagerie par tomographie spectrale à rayons X (10), comprenant :
- une unité d'imagerie par tomographie spectrale à rayons X (20) ;
- une unité de traitement (30) ; et
- une unité de sortie (40) ;
dans lequel l'unité d'imagerie de tomographie spectrale à rayons X comprend un tube à rayons X (22) et un détecteur à rayons X à double couche (24), et dans lequel une partie du corps d'un sujet à examiner peut être située entre le tube à rayons X et le détecteur à rayons X à double couche ;
dans lequel l'unité de traitement est configurée pour acquérir deux scanogrammes 2D de la partie du corps à différents angles de projection ou un scanogramme 3D de la partie du corps ;
dans lequel l'unité de traitement est configurée pour utiliser les profils d'épaisseur et d'atténuation de la partie du corps à travers différentes projections des deux scanogrammes 2D ou du scanogramme 3D, dans lequel les profils permettent de déterminer une tension pour le tube à rayons X pour chaque acquisition d'une pluralité d'acquisitions d'un balayage global mis en œuvre ;
dans lequel l'unité de traitement est configurée pour commander l'unité d'imagerie de tomographie spectrale à rayons X afin d'effectuer le balayage global mis en œuvre de la partie du corps, dans lequel la commande comprend la commande du tube à rayons X pour qu'il fonctionne à la tension du tube à rayons X déterminée pour chaque acquisition de la pluralité d'acquisitions aux différents angles de projection du balayage global mis en œuvre ;
dans lequel l'unité de traitement est configurée pour recevoir des données du détecteur à rayons X double couche pour chaque acquisition du balayage global mis en œuvre ;
dans lequel l'unité de traitement est configurée pour mettre en œuvre un algorithme d'apprentissage automatique afin de déterminer l'imagerie de décomposition des matériaux de la partie du corps, dans lequel la détermination comprend l'utilisation des données du détecteur à rayons X à double couche pour chaque acquisition du balayage global mis en œuvre et la tension du tube à rayons X déterminée pour chaque acquisition du balayage global mis en œuvre ; et
dans lequel l'unité de sortie est configurée pour produire l'imagerie de décomposition matérielle de la partie du corps.

2. Système selon la revendication 1, dans lequel les informations sur la taille du patient et les caractéristiques d'atténuation de la partie du corps pour chaque projection des différents angles de projection comprennent une épaisseur de la partie du corps pour chaque projection des différents angles de projection.

3. Système selon l'une quelconque des revendications 1-2, dans lequel les informations sur la taille du patient et les caractéristiques d'atténuation de la partie du corps pour chaque projection des différents angles de projection comprennent une atténuation des rayons X de la partie du corps pour chaque projection des différents angles de projection.

4. Système selon l'une quelconque des revendications 1-2, dans lequel la détermination de l'imagerie de décomposition matérielle de la partie du corps comprend l'utilisation de spectres de rayons X associés à chaque tension de tube à rayons X déterminée pour chaque acquisition du balayage global mis en œuvre.

5. Système selon la revendication 1, dans lequel les deux scanogrammes 2D de la partie du corps sont deux scanogrammes 2D orthogonaux de la partie du corps.

6. Système selon l'une quelconque des revendications 1-2, dans lequel le système comprend au moins une caméra visible ou infrarouge (50), dans lequel l'unité de traitement est configurée pour commander la au moins une caméra visible et/ou caméra infrarouge pour acquérir des données d'imagerie visibles et/ou IR de la partie du corps, dans lequel l'unité de traitement est configurée pour recevoir les données d'imagerie visibles et/ou infrarouges de la partie du corps, et dans lequel l'unité de traitement est en outre configurée pour déterminer les épaisseurs et les profils d'atténuation de la partie du corps pour chaque projection des différents angles de projection, sur la base des données d'imagerie visibles et/ou IR de la partie du corps.

7. Système selon l'une quelconque des revendications 1-2, dans lequel la détermination de la tension du tube à rayons X pour chaque acquisition de la pluralité d'acquisitions comprend une comparaison des informations correspondantes sur la taille du patient et les caractéristiques d'atténuation de la partie du corps pour la projection associée à cette acquisition avec des données de référence de différentes informations sur les parties du corps par rapport à différentes tensions du tube à rayons X.

8. Système selon l'une quelconque des revendications 1-2, dans lequel la détermination de la tension du tube à rayons X pour chaque acquisition de la pluralité d'acquisitions comprend une détermination d'une tension de tube à rayons X qui est déterminée pour donner une force de signal sensiblement équivalente dans la couche de détecteur supérieure et la couche de détecteur inférieure du détecteur à rayons X à double couche pour la taille du patient correspondante et les informations sur les caractéristiques d'atténuation sur la partie du corps pour la projection associée à cette acquisition.

9. Système selon l'une quelconque des revendications 1-2, dans lequel la détermination de la tension du tube à rayons X pour chaque acquisition de la pluralité d'acquisitions comprend une détermination d'une tension de tube à rayons X qui est déterminée pour donner un rapport signal/bruit maximal pour les données du détecteur à rayons X à double couche pour la taille du patient correspondante et les informations sur les caractéristiques d'atténuation sur la partie du corps pour la projection associée à cette acquisition.

10. Système selon l'une quelconque des revendications 1-2, dans lequel l'unité de traitement est configurée pour déterminer un courant pour le tube à rayons X pour chaque acquisition de la pluralité d'acquisitions, dans lequel la détermination pour chaque acquisition comprend l'utilisation des informations correspondantes sur la taille du patient et les caractéristiques d'atténuation de la partie du corps pour la projection associée à cette acquisition.

11. Système selon la revendication 12, dans lequel la détermination du courant du tube à rayons X pour chaque acquisition de la pluralité d'acquisitions comprend une détermination d'un courant de tube à rayons X déterminé pour donner lieu à une dose de rayons X sensiblement identique reçue par la partie du corps pour chaque acquisition.

12. Procédé d'imagerie par tomographie spectrale aux rayons X (100), comprenant :
a) la localisation (110) d'une partie du corps d'un sujet à examiner entre un tube à rayons X et un détecteur à rayons X à double couche d'une unité d'imagerie de tomographie spectrale à rayons X, et dans lequel l'unité d'imagerie de tomographie spectrale à rayons X est configurée pour acquérir deux scanogrammes 2D de la partie du corps comprenant une pluralité d'acquisitions à différents angles de projection ou un scanogramme 3D de la partie du corps ;
c) l'utilisation, par une unité de traitement, de profils d'épaisseur et d'atténuation de la partie du corps à travers différentes projections des deux scanogrammes 2D ou du scanogramme 3D, dans lequel les profils permettent de déterminer (120) par l'unité de traitement une tension pour le tube à rayons X pour chaque acquisition d'une pluralité d'acquisitions d'un balayage global mis en œuvre,
e) la commande (130) par l'unité de traitement l'unité d'imagerie de tomographie spectrale à rayons X pour effectuer un balayage global mis en œuvre de la partie du corps, dans lequel la commande comprend la commande du tube à rayons X pour qu'il fonctionne à la tension du tube à rayons X déterminée pour chaque acquisition de la pluralité d'acquisitions aux différents angles de projection du balayage global mis en œuvre ;
f) la réception (140) par l'unité de traitement des données du détecteur à rayons X double couche pour chaque acquisition du balayage global mis en œuvre ;
g) la mise en œuvre par l'unité de traitement d'un algorithme d'apprentissage automatique et la détermination (150) par l'algorithme d'apprentissage automatique de l'imagerie de décomposition des matériaux de la partie du corps, dans lequel la détermination comprend l'utilisation des données du détecteur à rayons X à double couche pour chaque acquisition du balayage global mis en œuvre et la tension du tube à rayons X déterminée pour chaque acquisition du balayage global mis en œuvre ; et
h) l'émission (160) par une unité de sortie de l'imagerie de décomposition matérielle de la partie du corps.

13. Élément de programme informatique pour commander un système selon l'une quelconque des revendications 1-2, qui, lorsqu'il est exécuté par un processeur, est configuré pour réaliser le procédé selon la revendication 12.
